# EUROPEAN PATENT APPLICATION

(11) **EP 3 679 976 A1**
(43) Date of publication of application: **15.07.2020**
(21) Application number: 18854968.7
(22) Date of filing: 31.07.2018
(51) Int. Cl.: A61M 25/10

(54) **CATHETER**

(30) Priority: 06.09.2017 JP 2017170895
(71) Applicant: Japan Lifeline Co., Ltd., Tokyo 140-0002 (JP)
(72) Inventor: MASUDA, Takuya, Tokyo 140-0002 (JP); HOSHIDA, Aki, Tokyo 140-0002 (JP)
(74) Representative: Grünecker Patent- und Rechtsanwälte PartG mbB
(86) International application number: PCT/JP2018/028593
(87) International publication number: WO 2019/049558

(57) **Abstract**

An object is to provide a novel temperature measuring catheter capable of planarly grasping a temperature distribution in an inner portion of a hollow organ, such as an esophagus, in a body and capable of reliably measuring the temperature of a region (region whose temperature is raised by ablation) that should be monitored. A catheter of the present invention includes: a catheter shaft (10) having a multi-lumen structure including a fluid-flowing lumen (11) for a fluid; a handle (20) connected to a proximal end side of the catheter shaft (10); a balloon (30) connected to a distal end side of the catheter shaft (10) and configured to be inflated by the fluid that flows through the fluid-flowing lumen (11), the balloon (30) being flat during inflation; and a plurality of temperature sensors (40) planarly disposed on one surface side of the balloon (30).

## Description

### Technical Field

The present invention relates to a catheter to be used for measuring an internal temperature of a hollow organ, such as an esophagus, in a body.

### Background Art

For example, to prevent an esophagus positioned near a left atrium from being overheated and causing esophageal fistula and the like in an ablation operation of the left atrium for treating atrial fibrillation, inserting a catheter for temperature measurement into an inner portion of the esophagus of a subject to be operated by a nasally approach and monitoring the temperature of the inner portion (inner wall) of the esophagus have been proposed (refer to, for example, PTL 1 presented below).

As a catheter for measuring the temperature of an inner portion of an esophagus, there is provided an electrode catheter (esophagus catheter) including a plurality of ring-shaped electrodes (electrodes for temperature measurement) mounted at a distal end portion of a catheter shaft so as to be away from each other, a plurality of temperature sensors (specifically, measuring junctions of a thermocouple) electrically connected to the ring-shaped electrode by being spot-welded to respective inner circumferential surfaces of these ring-shaped electrodes, and respective lead wires (specifically, metal wires of different types constituting the thermocouple) of these temperature sensors.

A plurality of side holes (through holes) are formed and arranged in a tube wall of the distal end portion of the catheter shaft constituting such an esophagus catheter in correspondence to positions at which the ring-shaped electrodes are mounted. The lead wires spot-welded to the inner circumferential surface of the ring-shaped electrodes and connected to the temperature sensors enter a lumen from the side holes formed in the tube wall of the catheter shaft, extend in the lumen and an inner portion of a control handle, and is connected to a connector.

During the ablation operation of the left atrium, when the temperature of the inner portion of the esophagus measured by any of the temperature sensors of such an esophagus catheter reaches a predetermined temperature (for example, 43°C), energization to an ablation catheter is shut off, thereby avoiding the esophagus from being overheated.

Meanwhile, the esophagus usually has a flat elliptical tube shape, and the internal temperature of the esophagus during an ablation operation of a left atrium is distributed not only in the length direction of the esophagus but also in the width direction thereof.

However, when an electrode catheter such as that described in PTL 1 is left in the inner portion of the esophagus, the plurality of ring-shaped electrodes (electrodes for temperature measurement) are disposed in the length direction of the esophagus, and it is thus not possible to measure the temperature distribution in the width direction of the esophagus.

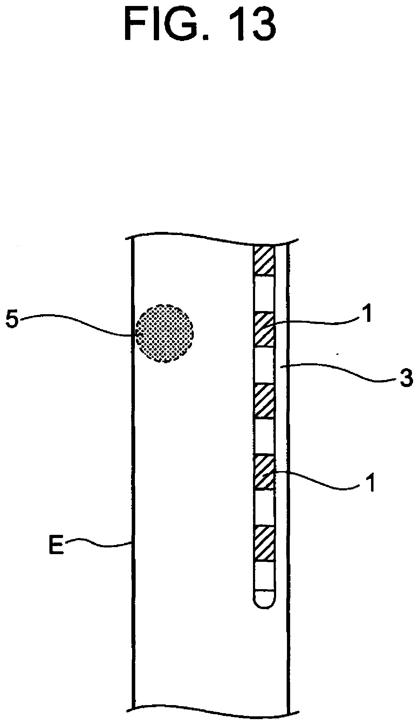

Thus, when a distal end part 3 of a catheter shaft at which ring-shaped electrodes 1 is mounted is left so as to be away from a region 5 whose temperature is raised by ablation (cauterization) in the width direction of an esophagus E as illustrated in Fig. 13, it is not possible to exactly detect an increase in the internal temperature of the esophagus E due to the ablation.

To address such a problem, as a temperature probe for monitoring the temperature of tissues in a body or a surface of an organ, there is proposed a probe including: a distal end part deformable into a meandering shape, in other words, a shaft having a deformable section that memorizes a shape meandering on a same plane; and a plurality of temperature sensors (ring-shaped electrodes) mounted at the distal end part of the shaft (refer to PTL 2 presented below).

However, such a temperature probe is also not capable of precisely measuring the temperature distribution in an esophagus in the width direction.

In other words, the plurality of temperature sensors are disposed planarly (dispersed in a substantially two-dimensional arrangement) when the distal end part of the shaft having the meandering shape is left in an esophagus, and, however, an arrangement interval of the temperature sensors is wide. Moreover, the temperature sensors adjacent to each other are away from each other not only in the width direction of the esophagus but also in the length direction of the esophagus.

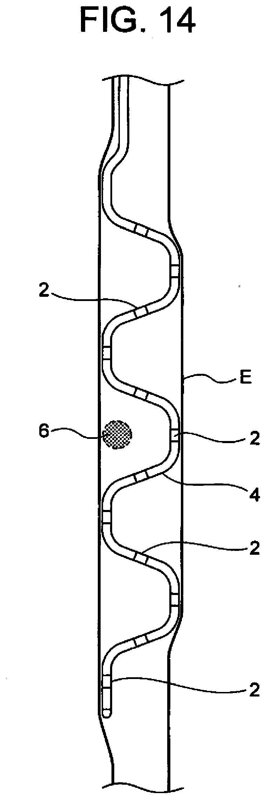

Thus, there is a case in which, as illustrated in Fig. 14, it is not possible to position ring-shaped electrodes 2 mounted at a distal end part 4 of the shaft, so as to be close to a region 6 whose temperature is raised by ablation. In such a state, it is not possible to exactly detect an increase in the temperature of the inner portion of the esophagus E due to the ablation.

In addition, it has been pointed out that a temperature probe such as that described in PTL 2 may increase a risk of the esophagus being overheated as a result of the distal end part expanding the esophagus and increasing a contact area with respect to a rear wall of the left atrium when the distal end part of the shaft left in the inner portion of the esophagus deforms (restores) into the meandering shape, which may lead to an increase of complications (refer to NPL 1 presented below).

Moreover, in an existing temperature measuring catheter in which a temperature sensor is spot welded to an inner circumferential surface of a ring-shaped electrode, the temperature of the inner circumferential surface of the ring-shaped electrode is measured as the temperature of an inner portion of an esophagus by the temperature sensor. However, there is a case in which it is not possible to quickly detect an actual change in the temperature of the inner portion of the esophagus because it takes a certain period of time for the temperature of the inner circumferential surface of the ring-shaped electrode to be raised.

In particular, due to a temperature difference present between an electrode part facing the ablation side and an electrode part on a side opposite to the ablation side, when the temperature sensor is positioned at the inner circumferential surface of the electrode part on the side opposite to the ablation side, the electrode part facing the ablation side may reach a temperature for which the energization should be shut off even when the temperature of the inner portion of the esophagus measured by this temperature sensor has not reached a temperature for which energization should be shut off, and the esophagus may be in an overheated state. In such a case, there is a risk of the esophagus being damaged.

### Citation List

### Patent Literature

PTL 1: Japanese Unexamined Patent Application Publication No. 2010-505592 (Claim 5)
PTL 2: Japanese Unexamined Patent Application Publication No. 2011-517417

### Non Patent Literature

NPL 1: Journal of Cardiovascular Electrophysiology Vol. 24, No. 9, September 2013

### Summary of Invention

### Technical Problem

The present invention is developed on the basis of the above circumstance.

A first object of the present invention is to provide a novel catheter for measuring an internal temperature of a hollow organ in a body.

A second object of the present invention is to provide a catheter capable of planarly grasping a temperature distribution in an inner portion of a hollow organ in a body and reliably measuring a temperature of a region that should be monitored.

A third object of the present invention is to provide a catheter capable of, during an ablation operation of a left atrium, planarly grasping a temperature distribution in an inner portion of an esophagus having a flat elliptical tube shape and reliably measuring a temperature of a region whose temperature is raised by ablation.

A fourth object of the present invention is to provide a temperature measuring catheter that does not expand an esophagus during an ablation operation of a left atrium.

A fifth object of the present invention is to provide a catheter capable of quickly measuring a temperature change of an inner portion of an esophagus, compared with an existing temperature measuring catheter including a ring-shaped electrode.

### Solution to Problem

(1) A catheter of the present invention is a catheter configured to measure an internal temperature of a hollow organ in a body, the catheter comprising:
   a catheter shaft having a multi-lumen structure including a fluid-flowing lumen for a fluid;
   a handle connected to a proximal end side of the catheter shaft;
   a balloon connected to a distal end side of the catheter shaft and configured to be inflated by the fluid that flows through the fluid-flowing lumen, the balloon being flat during inflation; and
   a plurality of temperature sensors planarly disposed at one surface side of the balloon.

   According to the catheter having such a configuration, it is possible to planarly grasp a temperature distribution in the inner portion of the hollow organ by inflating (inflation) the balloon in the inner portion of the hollow organ and measuring a temperature with the plurality of temperature sensors planarly disposed at the one surface side of the flat balloon, and it is possible to reliably measure the temperature of a region that should be monitored, with any one of or two or more of the plurality of planarly disposed temperature sensors.
   In addition, according to the catheter having such a configuration, it is possible to planarly grasp (grasp a length-direction temperature distribution and a width-direction temperature distribution in the esophagus) the temperature distribution in the inner portion of the esophagus by inflating the balloon in the inner portion of the esophagus that has a flat elliptical tube shape and measuring a temperature with the plurality of temperature sensors planarly disposed on the one surface side of the flat balloon, and it is possible to reliably measure the temperature of a region (region whose temperature should be monitored) whose temperature is raised by ablation, with any one of or two or more of the plurality of planarly disposed temperature sensors.
   In addition, due to the balloon that constitutes the catheter being flat during inflation, it is possible to prevent the inner wall of the esophagus from being pressed in the direction in which the left atrium is positioned by one surface of the inflated balloon.
   Moreover, by contracting (deflating) the balloon during the ablation operation of the left atrium, it is possible to completely avoid the inner wall of the esophagus from being pressed in the direction in which the left atrium is positioned by one surface of the balloon.
   Moreover, by contracting (deflating) the balloon during the ablation operation of the left atrium, it is also possible to completely avoid the esophagus from being expanded in the width direction by the balloon.
   In addition, the temperature sensors constituting the catheter are disposed on the one surface side of the balloon, and no ring-shaped electrode (electrode for temperature measurement) whose temperature rises and falls by absorbing and releasing the heat of ablation is interposed between the region whose temperature should be measured and the temperature sensors; it is thus possible to quickly measure a temperature change in the inner portion of the esophagus, compared with an existing temperature measuring catheter that includes such a ring-shaped electrode.
(2) In the catheter of the present invention, it is preferable that, at the one surface side of the balloon, a plurality of temperature-sensor groups be arranged in a width direction of the balloon, the plurality of temperature-sensor groups being constituted by the plurality of temperature sensors disposed in a length direction (direction that coincides with the axial direction of the catheter shaft) of the balloon.
   According to the catheter having such a configuration, it is possible to measure a temperature distribution in the length direction of the hollow organ with the plurality of temperature sensors belonging to respective temperature-sensor groups.
   In addition, it is possible to measure a temperature distribution in the width direction of the hollow organ with the plurality of temperature sensors belonging to the temperature-sensor groups that differ from each other and whose positions in the length direction of the balloon are identical or close to each other.
   As a result, it is possible to planarly grasp the temperature distribution in the inner portion of the hollow organ.
(3) In the catheter of the present invention, it is preferable that the balloon be constituted by a first sheet forming one surface and a second sheet forming another surface, the first sheet and the second sheet being partially fused with each other thereby forming a fluid housing space during inflation, and
   the plurality of temperature sensors be embedded in the first sheet.
   According to the catheter having such a configuration, it is possible to form a fluid housing space for a fluid at a non-fused part between the first sheet and the second sheet, the non-fused part being partitioned by fused parts between the first sheet and the second sheet. In addition, it is possible to planarly dispose these temperature sensors at one surface side of the balloon by embedding the plurality of temperature sensors in the first sheet.
(4) In the catheter described in (3), it is preferable that
   the first sheet be a flat sheet,
   the second sheet have a protruding portion that is not fused with the first sheet, and
   the first sheet and the protruding portion of the second sheet form the fluid housing space during inflation.
   According to the catheter having such a configuration, the one surface side of the balloon at which the plurality of temperature sensors are disposed is enabled to be flat, and, consequently, it is possible to measure a more precise temperature distribution.
(5) In the catheter described in (4), it is preferable that
   as the fluid housing space during inflation, the balloon include
   a first chamber extending in an axial direction of the catheter shaft,
   a second chamber extending parallel to the first chamber on one side of the first chamber with a space therebetween,
   a third chamber extending parallel to the first chamber on the other side of the first chamber with a space therebetween,
   a communication passage that causes the first chamber and the second chamber to communicate with each other, and
   a communication passage that causes the first chamber and the third chamber to communicate with each other,
   a distal end part of the catheter shaft be inserted into or inserted through the first chamber, and
   the catheter shaft have a distal end opening or a side hole for supplying the fluid in the fluid-flowing lumen into the first chamber.
   According to the catheter having such a configuration, the fluid is supplied from the distal end opening or the side hole of the catheter shaft into the first chamber of the balloon, the fluid supplied into the first chamber flows through the communication passages and is supplied into the second chamber and the third chamber, and the balloon is thereby inflated.
   Here, the first chamber, the second chamber, and the third chamber, which are the fluid housing spaces, extend parallel to each other in the axial direction of the catheter shaft. A fused part between the first sheet and the second sheet is formed between the first chamber and the second chamber, and a fused part between the first sheet and the second sheet is also formed between the first chamber and the third chamber. Consequently, it is possible to sufficiently ensure the flatness (in particular, the flatness of the balloon in the cross-sectional view) of the balloon during inflation.
(6) in the catheter described in (5), it is preferable that
   the communication passages extending from the first chamber on two sides (one side and the other side) thereof so as to cause the first chamber to communicate with the second chamber and the third chamber be a plurality of communication passages formed in the axial direction of the catheter shaft.
   According to the catheter having such a configuration, due to the communication passages extending from the first chamber on the two sides thereof being the plurality of communication passages formed in the axial direction of the catheter shaft, it is possible to further improve the flatness (in particular, the flatness of the balloon in the longitudinal-sectional view) of the balloon during inflation.
(7) In the catheter described in (6), it is preferable that
   the communication passages extending from the first chamber on the two sides thereof be formed in the axial direction of the catheter shaft at an equal interval.
   According to the catheter having such a configuration, the fluid housing spaces are formed in a grid shape, and the fused parts between the first sheet and the second sheet are planarly arranged. Consequently, the flatness of the balloon during inflation is particularly excellent, and it is possible to use the communication passages that are visually recognized on an X-ray image as scales indicating length.
(8) In the catheter of the present invention, it is preferable that the catheter include a flexible part at a distal end of the catheter shaft, and the distal end be deflectable.
(9) In the catheter of the present invention, it is preferable that the catheter be configured to be used for measuring an internal temperature of an esophagus during an ablation operation of a left atrium.

### Advantageous Effects of Invention

According to the catheter of the present invention, it is possible to planarly grasp a temperature distribution in an inner portion of a hollow organ in a body and reliably measure a temperature of a region that should be monitored.

According to the catheter of the present invention, it is possible, during an ablation operation of a left atrium, to planarly grasp a temperature distribution in an inner portion of an esophagus having a flat elliptical tube shape and reliably measure a temperature of a region whose temperature is raised by ablation.

According to the catheter of the present invention, it is possible, during an ablation operation of a left atrium, to avoid an esophagus having a flat elliptical tube shape from being expanded by a balloon, which is a portion at which temperature sensors are mounted. In addition, it is also possible to avoid an inner wall of the esophagus from being pressed in a direction in which the left atrium is positioned by one surface of the balloon.

According to the catheter of the present invention, it is possible to quickly measure a temperature change of an inner portion of an esophagus, compared with an existing temperature measuring catheter including a ring-shaped electrode.

### Brief Description of Drawings

[Fig. 1] Fig. 1 is a front view of a temperature measuring catheter according to an embodiment of the present invention.
[Fig. 2] Fig. 2 is a front view (partial enlarged front view of Fig. 1) illustrating a distal end portion of the temperature measuring catheter illustrated in Fig. 1.
[Fig. 3] Fig. 3 is a side view illustrating the distal end portion of the temperature measuring catheter illustrated in Fig. 1.
[Fig. 4] Fig. 4 is a rear view illustrating the distal end portion of the temperature measuring catheter illustrated in Fig. 1.
[Fig. 5A] Fig. 5A is a sectional view along VA-VA of Fig. 2.
[Fig. 5B] Fig. 5B is a sectional view along VB-VB of Fig. 2.
[Fig. 5C] Fig. 5C is a sectional view along VC-VC of Fig. 2.
[Fig. 6] Fig. 6 is an enlarged view (sectional view of a catheter shaft) of the part VI of Fig. 5C.
[Fig. 7] Fig. 7 is a sectional view (sectional view of the catheter shaft) along VII-VII of Fig. 2.
[Fig. 8] Fig. 8 is an illustration illustrating an example of the arrangement of temperature sensors at a balloon.
[Fig. 9] Fig. 9 is a graph showing a test result of responsiveness to a temperature change in a temperature measuring catheter.
[Fig. 10] Fig. 10 is a front view illustrating a distal end portion of a temperature measuring catheter according to another embodiment of the present invention.
[Fig. 11] Fig. 11 is a rear view illustrating the distal end portion of the temperature measuring catheter illustrated in Fig. 10.
[Fig. 12] Fig. 12 is a sectional view along XII-XII of Fig. 10.
[Fig. 13] Fig. 13 is an illustration schematically illustrating a state in which a distal end part of an existing publicly-known temperature measuring catheter is left in an esophagus.
[Fig. 14] Fig. 14 is an illustration schematically illustrating a state in which a distal end part of an existing publicly-known temperature measuring catheter is left in an esophagus.

### Description of Embodiments

### <First Embodiment>

Hereinafter, an embodiment of a catheter (temperature measuring catheter) of the present invention will be described with reference to the drawings.

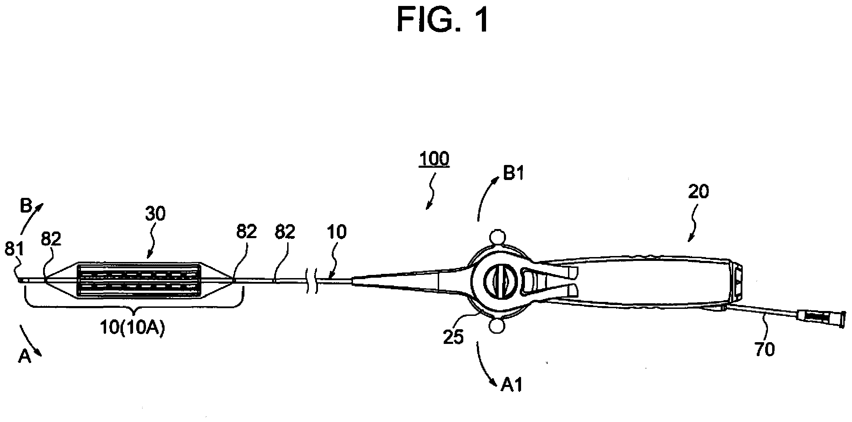

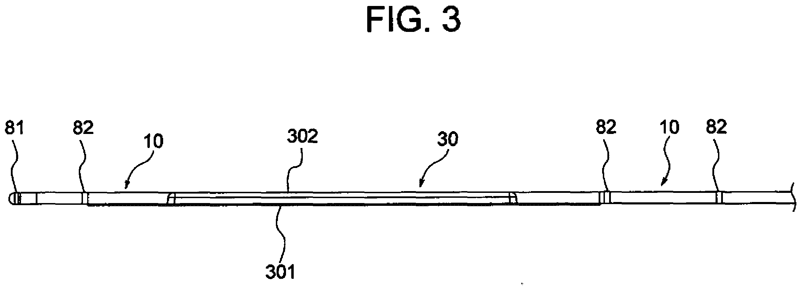

A temperature measuring catheter 100 of the present embodiment illustrated in Fig. 1 to Fig. 8 is a catheter for measuring an internal temperature of an esophagus during an ablation operation of a left atrium.

The temperature measuring catheter 100 includes: a catheter shaft 10 having a distal end flexible part 10A; a control handle 20 connected to the proximal end side of the catheter shaft 10; a balloon 30 connected to a distal end side of the catheter shaft 10; temperature sensors 40 (401 to 424) planarly disposed at one surface side of the balloon 30; lead wires 50 connected to respective temperature sensors 40; a first operation wire 61 for bending the distal end flexible part 10A of the catheter shaft 10 in a first direction (direction indicated by the arrow A in Fig. 1); a second operation wire 62 for bending the distal end flexible part 10A of the catheter shaft 10 in a second direction (direction indicated by the arrow B in Fig. 1); and a fluid injection tube 70.

In Figs. 1 to 4, the reference sign 81 denotes a distal end tip, and the reference sign 82 denotes an electrode for pacing a heart.

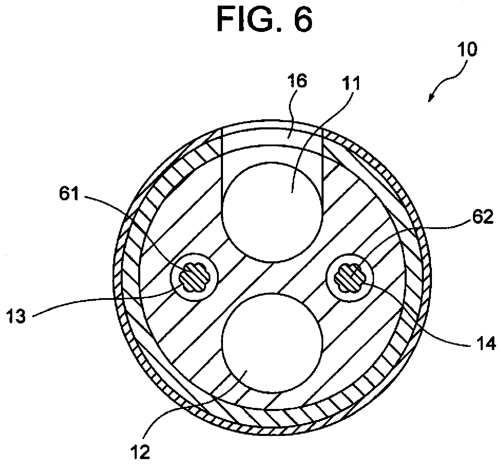

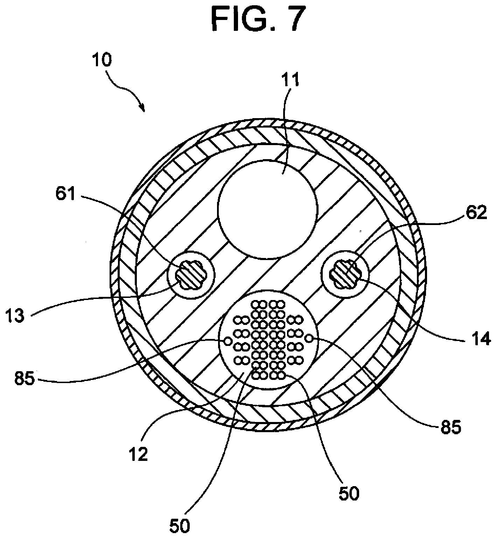

As illustrated in Fig. 6 and Fig. 7, in the catheter shaft 10 constituting the temperature measuring catheter 100, there are formed: a fluid-flowing lumen (so-called inflation lumen) 11 through which a fluid for inflating the balloon 30 flows; a lead insertion lumen 12 through which the lead wires 50 of the temperature sensors 40 and lead wires 85 of the electrodes 82 are inserted; a wire insertion lumen 13 through which the first operation wire 61 is inserted; and a wire insertion lumen 14 through which the second operation wire 62 is inserted.

The fluid that flows through the fluid-flowing lumen 11 is supplied from the fluid injection tube 70 to the fluid-flowing lumen 11 via an inner portion of the control handle 20 and flows from a side hole 16 opening at an outer circumferential surface of a distal end part (the distal end flexible part 10A) of the catheter shaft 10 into an inner portion (later-described first chamber 31) of the balloon 30.

Here, as the fluid, physiological saline can be exemplified.

The outer diameter of the catheter shaft 10 is normally 1.0 to 4.0 mm.

The length of the catheter shaft 10 is normally 300 to 1500 mm.

As a constituent material of the catheter shaft 10, thermoplastic resins, such as polyamide, polyether polyamide, polyurethane, polyether block amide (PEBAX) (registered trademark), nylon, and the like, can be listed, and, among these resins, the PEBAX is preferable.

The control handle 20 is connected to the proximal end side of the catheter shaft 10.

In the inner portion of the control handle 20 constituting the temperature measuring catheter 100, a connector including a plurality of terminals is disposed. The proximal ends of the lead wires 50 of the temperature sensors 40 and the proximal ends of the lead wires 85 of the electrodes 82 are connected to the terminals of the connector.

In addition, on the control handle 20, a grip 25 for performing an operation of bending a distal end part of the catheter shaft 10 is mounted.

The balloon 30 is connected to the distal end side of the catheter shaft 10.

The balloon 30 constituting the temperature measuring catheter 100 is inflated by the fluid that flows through the fluid-flowing lumen 11 of the catheter shaft 10 and has a flat shape even during inflation. The balloon 30 is constituted by a first sheet 301 forming one surface thereof and a second sheet 302 forming another surface thereof, the first sheet 301 and the second sheet 302 being partially fused with each other.

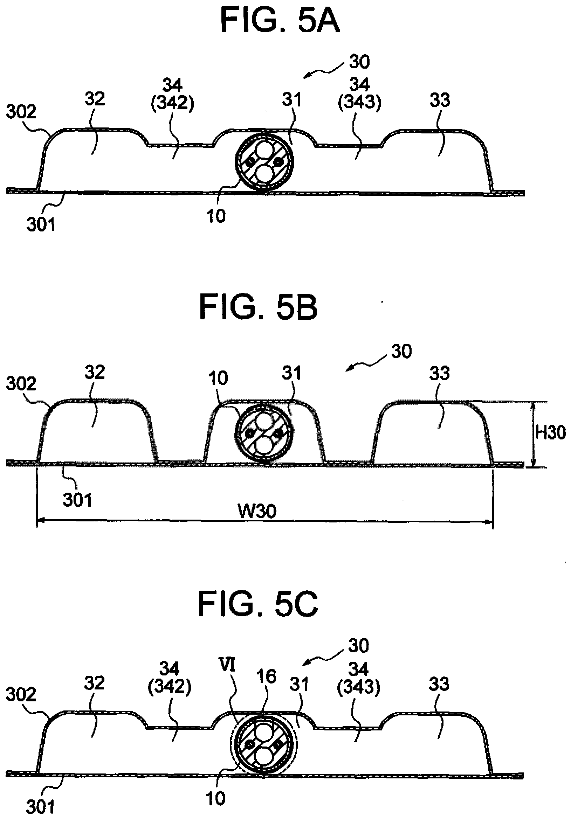

As illustrated in Fig. 5A to Fig. 5C, the first sheet 301 is a flat sheet, and the second sheet 302 has protruding portions that are not fused with the first sheet 301.

The first sheet 301 and the protruding portions of the second sheet 302 form fluid housing spaces during inflation.

Specifically, there are formed: the first chamber 31 extending in an axial direction of the catheter shaft 10; a second chamber 32 extending parallel to the first chamber 31 on one side of the first chamber 31 with a space therebetween; a third chamber 33 extending parallel to the first chamber 31 on the other side of the first chamber 31 with a space therebetween; communication passages 34 (342) that cause the first chamber 31 and the second chamber 32 to communicate with each other; and communication passages 34 (343) that cause the first chamber 31 and the third chamber 33 to communicate with each other.

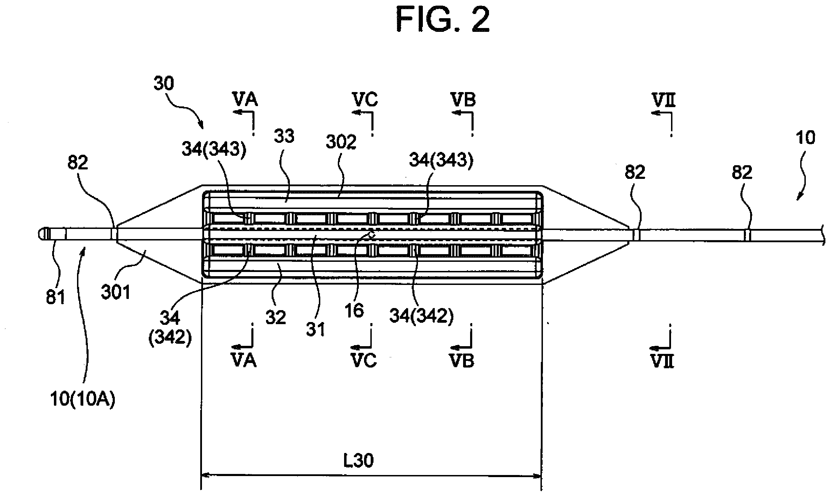

As illustrated in Fig. 1 and Fig. 2, the communication passages 34 (communication passages 342 and communication passages 343) extending from the first chamber 31 on two sides thereof are formed at an equal interval in the axial direction of the catheter shaft 10.

As the length (L30 indicated in Fig. 2) of the balloon 30, 30 to 100 mm is preferable, and one suitable example thereof is 60 mm.

The width (W30 indicated in Fig. 5B) of the balloon 30 is determined in consideration of the width of an esophagus having a shape of a flat elliptical tube (inner diameter (major diameter) of the elliptical tube). As the width (W30), 10 to 30 mm is preferable, and one suitable example thereof is 20 mm.

The thickness (H30 indicated in Fig. 5B) of the balloon 30 during inflation is determined in consideration of the inner diameter (minor diameter) of the elliptical tube. As the thickness (H30), 1 to 5 mm is preferable, and one suitable example thereof is 2 mm.

As each of the thicknesses of the first sheet 301 and the second sheet 302 constituting the balloon 30, 50 to 120 µm is preferable, and one suitable example thereof is 80 µm.

As a constituent material of the balloon 30, resins similar to the constituent material of the catheter shaft 10 are usable. Among these resins, polyurethane is preferable.

The balloon 30 is flat even during inflation. A ratio of the thickness thereof to the width thereof (H30/W30) is preferably 0.1 to 0.17, and one suitable example thereof is 0.1 (2 mm/ 20 mm).

As illustrated in Fig. 1 and Fig. 2, the distal end part (distal end flexible part 10A) of the catheter shaft 10 is inserted through the first chamber 31 and extends from the distal end of the first chamber 31 (balloon 30) in a state in which liquid tightness of the first chamber 31 is ensured.

The fluid that flows into the first chamber 31 from the side hole 16 opening at the outer circumferential surface of the distal end part of the catheter shaft 10 flows through the communication passages 34 (342 and 343) and flows into the second chamber 32 and the third chamber 33, all of the housing spaces (first chamber 31, second chamber 32, third chamber 33, and communication passages 34) are filled with the fluid, and the balloon 30 is thereby inflated.

In the balloon 30, the fluid housing spaces (first chamber 31, second chamber 32, third chamber 33, and communication passages 34) are formed in a grid shape, and fused parts between the first sheet 301 and the second sheet 302 are planarly arranged. Therefore, the balloon 30 is excellent in particular in flatness during inflation.

According to the balloon 30 thus excellent in flatness, it is possible to reliably cause the flat shape of the balloon 30 to coincide with the flat elliptical tube shape of an esophagus during inflation and after contraction of the balloon 30.

Due to the communication passages 34 (communication passages 342 and communication passages 343) that extend from the first chamber 31 on two sides thereof being formed in the axial direction of the catheter shaft 10 at an equal interval, the communication passages 34 that are visually recognized on an X-ray image are enabled to be used as scales indicating length.

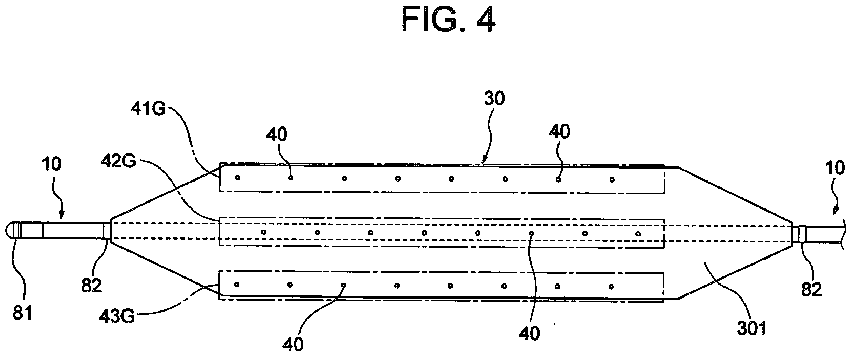

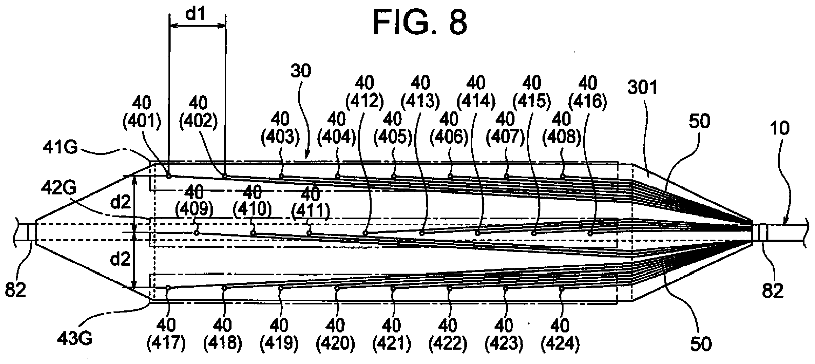

As illustrated in Fig. 4 and Fig. 8, the temperature sensors 40 (401 to 424) are planarly disposed on the one surface side of the balloon 30.

The temperature sensors 40 (401 to 424) constituting the temperature measuring catheter 100 are constituted by, for example, measuring junctions of a thermocouple and embedded in the first sheet 301 forming one surface of the balloon 30 (the temperature sensors 40 embedded in the first sheet 301 are indicated by solid lines in Fig. 4 and Fig. 8: in addition, illustrations of the temperature sensors 40 that appear at the cross section of the first sheet 301 are omitted in Fig. 5A to Fig. 5C.).

Consequently, it is possible to dispose the temperature sensors 40 (401 to 424) on the one surface side of the balloon 30. In addition, it is possible to measure a highly precise temperature distribution because the first sheet 301 in which the temperature sensors 40 are embedded is a flat sheet.

As illustrated in Fig. 8, on the one surface side of the balloon 30, the temperature sensors 40 (401 to 408) disposed in the length direction of the balloon 30 (axial direction of the catheter shaft 10) at an equal interval constitute a first temperature-sensor group 41G, the temperature sensors 40 (409 to 416) disposed in the aforementioned direction at an equal interval constitute a second temperature-sensor group 42G, and the temperature sensors 40 (417 to 424) disposed in the aforementioned direction at an equal interval constitute a third temperature-sensor group 43G.

Here, as a clearance (d1 indicated in Fig. 8) between the temperature sensors 40 adjacent to each other in the length direction of the balloon 30, 3.5 to 11.7 mm is preferable, and one suitable example thereof is 7.0 mm.

The first temperature-sensor group 41G constituted by the temperature sensors 401 to 408, the second temperature-sensor group 42G constituted by the temperature sensors 409 to 416, and the third temperature-sensor group 43G constituted by the temperature sensors 417 to 424 are arranged in the width direction of the balloon 30 at an equal interval.

Here, as a clearance (d2 indicated in Fig. 8) between the temperature-sensor groups adjacent to each other in the width direction of the balloon 30, 3 to 13 mm is preferable, and one suitable example thereof is 7.5 mm.

The position of each of the temperature sensors 40 (401 to 408) that constitute the first temperature-sensor group 41G in the length direction of the balloon 30 and the position of each of the temperature sensors 40 (417 to 424) that constitute the third temperature-sensor group 43G in the length direction of the balloon 30 coincide with each other.

Meanwhile, the position of each of the temperature sensors 40 (409 to 416) that constitute the second temperature-sensor group 42G in the length direction of the balloon 30 is shifted by (d1/2) toward the proximal end side from the position of each of the temperature sensors that constitute the first temperature-sensor group 41G or the third temperature-sensor group 43G in the length direction of the balloon 30.

By disposing the temperature sensors 40 as illustrated in Fig. 8, it is possible to measure a temperature distribution in the length direction of an esophagus with the temperature sensors 40 belonging to a respective one of the first temperature-sensor group 41G, the second temperature-sensor group 42G, and the third temperature-sensor group 43G.

In addition, it is possible to measure a temperature distribution in the width direction of the esophagus with a plurality of the temperature sensors 40 (one example is the temperature sensor 405, the temperature sensor 412 and/or the temperature sensor 413, and the temperature sensor 421) belonging to the temperature-sensor groups that differ from each other and whose positions in the length direction of the balloon 30 are identical or close to each other.

Moreover, by concurrently measuring a temperature distribution in the length direction and the width direction of the esophagus, it is possible to planarly grasp the temperature distribution in an inner portion of the esophagus.

In addition, as a result of the position of each of the temperature sensors 40 that constitute the second temperature-sensor group 42G in the length direction of the balloon 30 being shifted by (d1/2) toward the proximal end side from the position of each of the temperature sensors that constitute the first temperature-sensor group 41G or the third temperature-sensor group 43G in the length direction of the balloon 30, it is possible to more precisely measure a temperature distribution in the length direction of the esophagus.

As illustrated in Fig. 8, the lead wires 50 are connected to each of the temperature sensors 40 (401 to 424).

The lead wires 50 are constituted by, for example, metal wires of different types constituting a thermocouple that uses each of the temperature sensors 40 (401 to 424) as a measuring junction.

These lead wires 50 extend in the proximal end direction in a state of being embedded in the first sheet 301 constituting the balloon 30 and enters the lead insertion lumen 12 in the vicinity of the proximal end of the first sheet 301 from a non-illustrated side hole formed at the outer circumferential surface of the catheter shaft 10.

In Fig. 8, the lead wires 50 embedded in the first sheet 301 are indicated by solid lines. In Fig. 4 and Fig. 5A to Fig. 5C, an illustration of the lead wires 50 is omitted.

The lead wires 50 that have entered the lead insertion lumen 12 extend, together with the lead wires 85 of the electrodes 82 for pacing, in the lead insertion lumen 12 and the inner portion of the control handle 20 and are connected to the connector.

In the temperature measuring catheter 100 of the present embodiment, the distal end thereof is deflectable by pulling the first operation wire 61 and the second operation wire 62.

Respective distal ends of the first operation wire 61 and the second operation wire 62 are fixed to the distal end part (slightly shifted toward the proximal end side from the position of the proximal end of the balloon 30) of the catheter shaft 10.

Meanwhile, respective rear ends of the first operation wire 61 and the second operation wire 62 are connected to the grip 25 of the control handle 20.

The distal end part (distal end flexible part 10A) of the catheter shaft 10 is bendable in the first direction (direction indicated by the arrow A of Fig. 1) by rotating the grip 25 of the control handle 20 in a direction indicated by the arrow A1 of Fig. 1 and thereby pulling the first operation wire 61.

The distal end part of the catheter shaft 10 is bendable in the second direction (direction indicated by the arrow B of Fig. 1) by rotating the grip 25 of the control handle 20 in a direction indicated by the arrow B1 of Fig. 1 and thereby pulling the second operation wire 62.

During an ablation operation of a left atrium, an internal temperature of an esophagus can be measured as described below with the temperature measuring catheter 100 of the present embodiment.

First, the temperature measuring catheter 100 is inserted into an inner portion of an esophagus of a subject to be operated by a nasally approach, and the balloon 30 mounted at the distal end side of the catheter shaft 10 is left at a region whose temperature should be monitored. Here, the balloon 30 during the insertion is in a state of being wound (wrapped) around the distal end part of the catheter shaft 10.

Next, a fluid (physiological saline) is supplied from the fluid injection tube 70 into the fluid-flowing lumen 11 of the catheter shaft 10. The fluid supplied into the fluid-flowing lumen 11 flows into the first chamber 31 of the balloon 30 from the side hole 16 opening at the outer circumferential surface of the distal end part of the catheter shaft 10, flows through the communication passages 34 (communication passages 342 and communication passages 343), and also flows into the second chamber 32 and the third chamber 33. As a result of the fluid being thus housed in the first chamber 31, the second chamber 32, the third chamber 33, and the communication passages 34, the balloon 30 in a wrapped state is developed and inflated into a flat shape.

The balloon 30 after the inflation is left such that the width direction thereof coincides with the width direction of the esophagus and that the one surface side of the balloon 30 is in contact with or face the inner wall of the esophagus on the left atrium side.

Here, when the esophagus is expanded by the inflated balloon 30 or the inner wall of the esophagus is pressed in a direction in which the left atrium is positioned by one surface of the inflated balloon 30, these states can be resolved by discharging part or all of the fluid housed in the fluid housing spaces (first chamber 31, second chamber 32, third chamber 33, and communication passages 34) to thereby contract the balloon 30.

Next, the temperature of the inner portion of the esophagus is measured with the temperature sensors 40 (401 to 424) disposed on the one surface side of the balloon 30 concurrently, and a temperature distribution is planarly grasped.

When the temperature of the inner portion of the esophagus measured by any of the temperature sensors reaches a predetermined temperature (for example, 43°C), ablation is stopped according to a routine procedure by shutting off energization to the ablation catheter.

According to the temperature measuring catheter 100 of the present embodiment, it is possible, during an ablation operation of a left atrium, to planarly grasp a temperature distribution in an inner portion of an esophagus having a flat elliptical tube shape by measuring a temperature with the temperature sensors 40 (401 to 424) planarly disposed at the one surface side of the balloon 30, and it is possible to reliably measure the temperature of a region (region whose temperature should be monitored) whose temperature is raised by ablation with the any one of or two or more of the temperature sensors 40.

In addition, due to the balloon 30 that constitutes the temperature measuring catheter 100 being flat even during inflation, it is possible during an ablation operation of a left atrium to prevent an inner wall of an esophagus from being pressed in a direction in which the left atrium is positioned by one surface of the inflated balloon 30.

In addition, it is possible by contracting the balloon 30 during the ablation operation of the left atrium to reliably avoid the inner wall of the esophagus from being pressed in the direction in which the left atrium is positioned by the one surface of the balloon 30.

In addition, it is possible by contracting the balloon 30 during the ablation operation of the left atrium to reliably avoid an esophagus from being expanded by the balloon 30.

In addition, flatness of the balloon 30 during inflation is particularly excellent because the fluid housing spaces (first chamber 31, second chamber 32, third chamber 33, and communication passages 34) of the balloon 30 constituting the temperature measuring catheter 100 are formed in a grid shape, and the fused parts between the first sheet 301 and the second sheet 302 are planarly arranged.

In addition, due to the communication passages 34 (communication passages 342 and communication passages 343) extending from the first chamber 31 on two sides thereof being formed in the axial direction of the catheter shaft 10 at an equal interval, it is possible to use the communication passages 34 visually recognized on an X-ray image as scales indicating length.

Moreover, in the temperature measuring catheter 100 of the present embodiment, no ring-shaped electrode (electrode for temperature measurement) is interposed between a region whose temperature should be measured and the temperature sensors 40 (401 to 424) disposed at the one surface side of the balloon 30 in the state of being embedded in the first sheet 301; it is thus possible to quickly measure a temperature change of an inner portion of an esophagus, compared with an existing temperature measuring catheter that includes such a ring-shaped electrode.

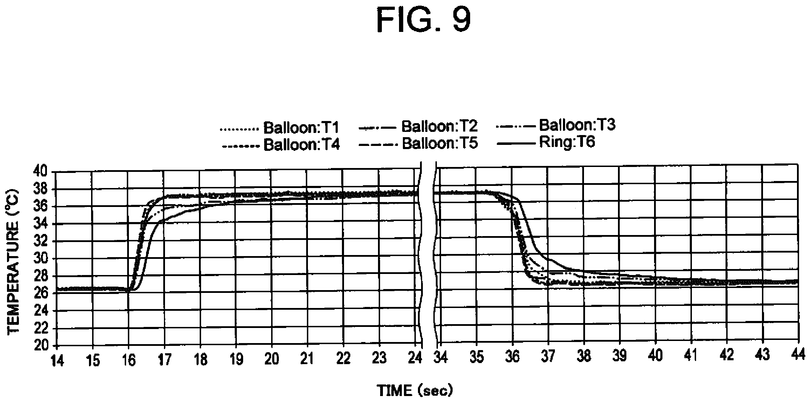

Fig. 9 indicates a test result of responsiveness to a temperature change in the temperature measuring catheter 100 of the present embodiment together with a test result of responsiveness to a temperature change in an existing temperature measuring catheter that includes a ring-shaped electrode.

Specifically, Fig. 9 is a graph showing results of tests in which mounting portions of the temperature sensors that had been immersed in water having a temperature of 26°C were immersed in water having a temperature of 37°C, responsiveness during a temperature rise was measured, the mounting portions of the temperature sensors were immersed in water having a temperature of 26°C, and responsiveness during a temperature fall was measured again.

In Fig. 9, "Balloon:T1", "Balloon:T2", "Balloon:T3", "Balloon:T4", and "Balloon:T5" each indicate a temperature change measured by the temperature sensor 404, the temperature sensor 405, the temperature sensor 412, the temperature sensor 413, or the temperature sensor 420 illustrated in Fig. 8, and "Ring:T6" indicates a temperature change measured by a temperature measuring catheter that includes a ring-shaped electrode.

Note that the tests were performed for the temperature measuring catheter 100 with the balloon 30, which is a mounting portion for the temperature sensors 40, in a contracted state.

As shown in Fig. 9, it is understood that the temperature measuring catheter 100 of the present embodiment is superior to the temperature measuring catheter that includes the ring-shaped electrode in terms of responsiveness (rising and falling) to a temperature change during both of a temperature rise and a temperature fall.

### <Second Embodiment>

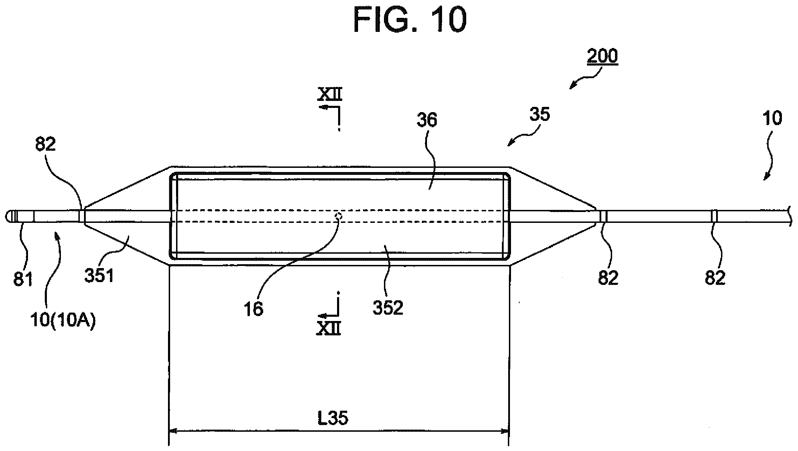

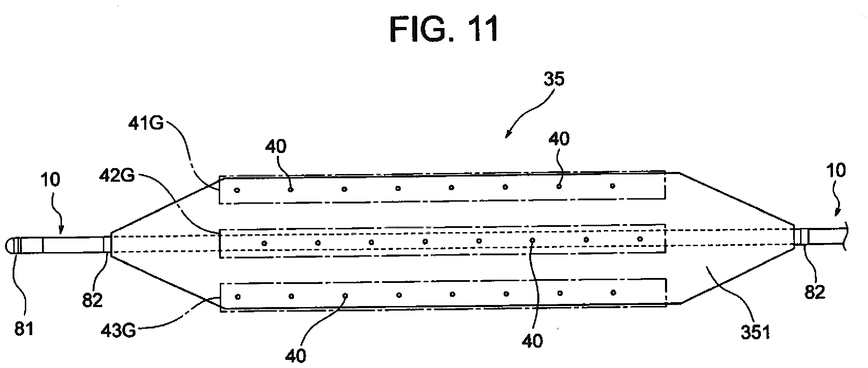

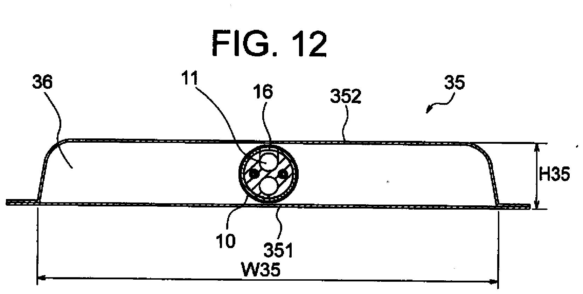

Fig. 10 and Fig. 11 each illustrate a distal end portion of a temperature measuring catheter according to a second embodiment, and Fig. 12 illustrates a sectional view along XII-XII of Fig. 10. In Fig. 10 to Fig. 12, for constituent components identical to those of the temperature measuring catheter 100 according to the first embodiment, identical reference sings are used.

In a temperature measuring catheter 200 of the present embodiment, the configuration of a balloon 35 connected to the distal end side of the catheter shaft 10 differs from that of the balloon 30 in the first embodiment.

The balloon 35 constituting the temperature measuring catheter 200 is inflated by a fluid that flows through the fluid-flowing lumen of the catheter shaft 10 and has a flat shape even during inflation.

The balloon 35 is constituted by a first sheet 351 forming one surface thereof and a second sheet 352 forming another surface thereof, the first sheet 351 and the second sheet 352 being partially fused with each other. Non-fused parts (non-fused parts partitioned by the fused parts) form fluid housing spaces during inflation.

As illustrated in Fig. 12, the first sheet 351 is a flat sheet, and the second sheet 352 has a protruding portion that is not fused with the first sheet 351.

The first sheet 351 and the protruding portion of the second sheet 352 form a fluid housing space 36 during inflation. In addition, the protruding portion of the second sheet 352 forms flat another surface of the balloon 35.

As the length (L35 indicated in Fig. 10) of the balloon 35, 30 to 100 mm is preferable, and one suitable example thereof is 60 mm.

The width (W35 indicated in Fig. 12) of the balloon 35 is determined in consideration of the width of an esophagus having a shape of a flat elliptical tube (inner diameter (major diameter) of the elliptical tube). As the width (W35), 10 to 30 mm is preferable, and one suitable example thereof is 20 mm.

The thickness (H35 indicated in Fig. 12) of the balloon 30 during inflation is determined in consideration of the inner diameter (minor diameter) of the elliptical tube. As the thickness (H35), 1 to 4 mm is preferable, and one suitable example thereof is 2 mm.

As each of the thicknesses of the first sheet 351 and the second sheet 352 constituting the balloon 35, 50 to 120 µm is preferable, and one suitable example thereof is 80 µm.

As a constituent material of the balloon 35, resins similar to the constituent material of the catheter shaft 10 are usable. Among these resins, polyurethane is preferable.

The balloon 35 is flat even during inflation. As a ratio of the thickness to the width thereof (H35/W35), 0.1 to 0.17 is preferable, and one suitable example thereof is 0.1 (2 mm / 20 mm) .

As illustrated in Fig. 10 and Fig. 11, the distal end part (distal end flexible part 10A) of the catheter shaft 10 is inserted through an inner portion (the fluid housing space 36) of the balloon 35 and extends from the distal end of the fluid housing space 36 in a state in which liquid tightness of the housing space 36 is ensured.

The fluid flows into the fluid housing space 36 from the side hole 16 opening at the outer circumferential surface of the distal end part of the catheter shaft 10, and the balloon 35 is thereby inflated.

According to the balloon 35 that is flat even during inflation, it is possible to cause the flat shape of the balloon 35 to coincide with the flat elliptical tube shape of the esophagus during inflation and after contraction of the balloon 35.

As illustrated in Fig. 11, a total of 24 of the temperature sensors 40 are planarly disposed on the one surface side of the balloon 35.

The temperature sensors 40 are embedded in the first sheet 351 forming the one surface of the balloon 35.

Consequently, it is possible to dispose the temperature sensors 40 on the one surface side of the balloon 35. In addition, it is possible to measure a highly precise temperature distribution because the first sheet 351 in which the temperature sensors 40 are embedded is a flat sheet.

The temperature sensors 40 embedded in the first sheet 351 are indicated by solid lines in Fig. 11. Illustrations of the temperature sensors 40 that appear at the cross section of the first sheet 351 are omitted in Fig. 12. In addition, illustrations of the lead wires are omitted in Fig. 11 and Fig. 12.

According to the temperature measuring catheter 200 of the present embodiment, it is possible during an ablation operation of a left atrium to planarly grasp a temperature distribution in an inner portion of an esophagus having a flat elliptical tube by measuring a temperature with the temperature sensors 40 planarly disposed on the one surface side of the balloon 35, and it is possible to reliably measure the temperature of a region (region whose temperature should be monitored) whose temperature is raised by ablation with any one of or two or more of the temperature sensors 40.

In addition, due to the balloon 35 that constitutes the temperature measuring catheter 200 being flat even during inflation, it is possible during an ablation operation of a left atrium to prevent an inner wall of an esophagus from being pressed in a direction in which the left atrium is positioned by one surface of the inflated balloon 35.

In addition, it is possible by contracting the balloon 35 during the ablation operation of the left atrium to reliably avoid the inner wall of the esophagus from being pressed in the direction in which the left atrium is positioned by the one surface of the balloon 35.

In addition, it is possible by contracting the balloon 35 during the ablation operation of the left atrium to reliably avoid the esophagus from being expanded by the balloon 35.

Moreover, in the temperature measuring catheter 200 of the present embodiment, no ring-shaped electrode (electrode for temperature measurement) is interposed between a region whose temperature should be measured and the temperature sensors 40 disposed on the one surface side of the balloon 35 in the state of being embedded in the first sheet 351; it is thus possible to quickly measure a temperature change of an inner portion of an esophagus, compared with an existing temperature measuring catheter that includes such a ring-shaped electrode.

Embodiments of the present invention have been described above; however, the present invention is not limited to these embodiments and can be variously modified.

For example, in the first embodiment, the distal end part of the catheter shaft inserted into the first chamber of the balloon may be in a state of being housed inside the first chamber without extending from the distal end of the first chamber. Similarly, in the second embodiment, the distal end part of the catheter shaft inserted into the inner portion (fluid housing space) of the balloon may be in a state of being housed in the inner portion of the balloon without extending from the distal end of the fluid housing space.

In addition, in the first embodiment, the communication passages that cause the first chamber and the second chamber to communicate with each other and the communication passages that cause the first chamber and the third chamber to communicate with each other may be each one communication passage.

In addition, the position of the temperature sensors constituting the first temperature-sensor group in the length direction of the balloon, the position of the temperature sensors constituting the second temperature-sensor group in the length direction of the balloon, and the position of the temperature sensors constituting the third temperature-sensor group in the length direction of the balloon may be coincide with each other.

### Reference Signs List

- 100: temperature measuring catheter
- 10: catheter shaft
- 11: fluid-flowing lumen
- 12: lead insertion lumen
- 13, 14: wire insertion lumen
- 16: side hole
- 20: control handle
- 25: grip
- 30: balloon
- 301: first sheet
- 302: second sheet
- 31: first chamber
- 32: second chamber
- 33: third chamber
- 34 (342, 343): communication passage
- 40 (401 to 424): temperature sensor
- 41G: first temperature-sensor group
- 42G: second temperature-sensor group
- 43G: third temperature-sensor group
- 50: lead wire of temperature sensor
- 61: first operation wire
- 62: second operation wire
- 70: fluid injection tube
- 81: distal end tip
- 82: electrode
- 85: lead wire of electrode
- 200: temperature measuring catheter
- 35: balloon
- 351: first sheet
- 352: second sheet
- 36: fluid housing space

## Claims

1. A catheter configured to measure an internal temperature of a hollow organ in a body, the catheter comprising:
a catheter shaft having a multi-lumen structure including a fluid-flowing lumen for a fluid;
a handle connected to a proximal end side of the catheter shaft;
a balloon connected to a distal end side of the catheter shaft and configured to be inflated by the fluid that flows through the fluid-flowing lumen, the balloon being flat during inflation; and
a plurality of temperature sensors planarly disposed at one surface side of the balloon.

2. The catheter according to claim 1, wherein, at the one surface side of the balloon, a plurality of temperature-sensor groups are arranged in a width direction of the balloon, the plurality of temperature-sensor groups being constituted by the plurality of temperature sensors disposed in a length direction of the balloon.

3. The catheter according to claim 1 or claim 2,
wherein the balloon is constituted by a first sheet forming one surface thereof and a second sheet forming another surface thereof, the first sheet and the second sheet being partially fused with each other, thereby forming a fluid housing space during inflation, and
wherein the plurality of temperature sensors are embedded in the first sheet.

4. The catheter according to claim 3,
wherein the first sheet is a flat sheet,
wherein the second sheet has a protruding portion that is not fused with the first sheet, and
wherein the first sheet and the protruding portion of the second sheet form the fluid housing space during inflation.

5. The catheter according to claim 4,
wherein, as the fluid housing space during inflation, the balloon includes
a first chamber extending in an axial direction of the catheter shaft,
a second chamber extending parallel to the first chamber on one side of the first chamber with a space therebetween,
a third chamber extending parallel to the first chamber on the other side of the first chamber with a space therebetween,
a communication passage that causes the first chamber and the second chamber to communicate with each other, and
a communication passage that causes the first chamber and the third chamber to communicate with each other,
wherein a distal end part of the catheter shaft is inserted into or inserted through the first chamber, and
wherein the catheter shaft has a distal end opening or a side hole for supplying the fluid in the fluid-flowing lumen into the first chamber.

6. The catheter according to claim 5, wherein the communication passages extending from the first chamber on two sides of the first chamber so as to cause the first chamber to communicate with the second chamber and the third chamber are a plurality of communication passages formed in the axial direction of the catheter shaft.

7. The catheter according to claim 6, wherein the communication passages extending from the first chamber on the two sides of the first chamber are formed in the axial direction of the catheter shaft at an equal interval.

8. The catheter according to any one of claims 1 to 7, comprising a flexible part at a distal end of the catheter shaft, the distal end being deflectable.

9. The catheter according to any one of claims 1 to 8, wherein the catheter is configured to be used for measuring an internal temperature of an esophagus during an ablation operation of a left atrium.
